# EUROPEAN PATENT APPLICATION

(11) **EP 1 743 907 A1**
(43) Date of publication of application: **17.01.2007**
(21) Application number: 06014064.7
(22) Date of filing: 06.07.2006
(51) Int. Cl.: C08B 37/00, A61K 47/48

(54) **Conjugate of cyclodextrin and poly(oxyethylene), and process for preparation thereof**

(30) Priority: 12.07.2005 KR 20050062830
(71) Applicant: Gwangju Institute of Science and Technology, Puk-gu Gwangju 500-712 (KR)
(72) Inventor: Geckeler, Kurt E., Puk-gu Gwangju 500-712 (KR); Choi, Sungho, Puk-gu Gwangju 500-712 (KR)
(74) Representative: Grünecker, Kinkeldey, Stockmair & Schwanhäusser Anwaltssozietät

(57) **Abstract**

A novel cyclodextrin conjugate having poly(oxyethylene) as a spacer is non-toxic and forms highly biocompatible supramolecular structures exhibiting significant inclusive property and structural diversity, and thus, can be beneficially employed in various fields such as drug delivery, food and flavors, cosmetics, packing, textiles, separation processes, environment protection, fermentation and catalysis.

## Description

### Field of the Invention

The present invention relates to a novel cyclodextrin conjugate and a process for the preparation thereof

### Description of the Prior Art

Cyclodextrin (CD) belongs to the cyclic oligosaccharide family composed α(1,4)-linked glucopyranose subunits, and it has a cage-like structure capable of forming inclusion complexes through host-guest interaction.

CD has, therefore, been widely used in industrial and analytical application on account of its capability to trap diverse guest molecules in its cavity and also because of its negligible cytotoxity. Accordingly, there have been numerous attempts to develop CD derivatives which can be employed in such fields as drug delivery, food and flavors, cosmetics, packaging, textiles, separation processes, environment protection, fermentation and catalysis (Martin del Valle, E. M., Process Biochemistry, 39, 1033-1046 (2004); Szejtli, J., J. Chem. Rev., 98, 1743-1753 (1988); Szejtli, J., J. Cyclodextrin Technology, 450 (1988); Szejtli, J. et al., Comprehensive Supramolecular Chemistry, 3, 693 (1996); Szejtli, J., J. Mater. Chem., 7, 575 (1997); and Jonathan W. Steed et al., Supramolecular Chemistry, 304-321 (2000)).

However, most of such CD derivatives are mono-CD conjugates having limited complex-forming capability. For the dimeric CD derivatives reported in the literable, there exist problems of requiring complicated synthetic procedures or linking spacers need in the preparation thereof being cytotoxic.

For example, the β-CD dimer, disclosed in Liu, Y et al., Org. Lett., 3(11), 1657-1660 (2001), has the structure of two CD molecules linked by a bipyridine spacer having hydrophilic ester moieties, and it is capable of quenching the fluorescence of Rhodamine B (RhB) because of the proximity of the carboxyl group of RhB to the spacer. However, this β-CD dimer is not biocompatible due to the cytotoxicity of the bipyridine moiety.

The present inventors have endeavored to develop a cyclodextrin conjugate that is free from the problems mentioned above, and have unexpectedly found that a cyclodextrin conjugate having a poly(oxyethylene) spacer can form biocompatible supramolecular structures which exhibit excellent properties for forming inclusive complexes.

### Summary of the Invention

Accordingly, it is a primary object of the present invention to provide a novel cyclodextrin conjugate capable of forming diverse supramolecular structures having satisfactory biocompatibility.

It is another object of the present invention to provide a process for the preparation of said cyclodextrin conjugate.

In accordance with one aspect of the present invention, there is provided a cyclodextrin (CD) conjugate having a poly(oxyethylene) (POE) moiety as a spacer.

In accordance with another aspect of the present invention, there is provided a method for preparing said CD conjugate comprising: (i) reacting poly(ethylene glycol) bis(carboxymethyl) ether with thionyl chloride or oxalyl chloride to obtain a poly(oxyethylene) bis-acid chloride derivative; and (ii) reacting the poly(oxyethylene) bis-acid chloride derivative obtained in step (i) with cyclodextrin.

In accordance with a further aspect of the present invention, there is provided a method for preparing said CD conjugate comprising reacting poly(ethylene glycol) bis(carboxymethyl) ether with cyclodextrin in the presence of a carboxyl group-activating coupling agent.

### Brief Description of the Drawings

The above and other objects and features of the present invention will become apparent from the following description of the invention, when taken in conjunction with the accompanying drawings, which respectively show:
FIGs. 1A and 1B: Molecular models of the inventive cyclodextrin (CD) conjugate having poly(oxyethylene) (POE) as a spacer attached to a CD molecule (monomeric CD) or a monomeric CD repeating unit of the inventive polymeric CD (FIG 1A), and the inventive CD conjugate having POE attached to two CD molecules (dimeric CD) (FIG 1B);
FIGs. 2A and 2B: Fourier transform-infrared spectrum (FT-IR) of poly(ethyleneglycol) bis(carboxymethyl) ether (FIG 2A) and poly(oxyethylene) bis-acid chloride derivative (FIG 2B);
FIG 3: Thermogravimetric analysis (TGA) curve of the inventive dimeric β-CD obtained in Example 1 (a), poly(oxyethylene) bis-acid chloride derivative (b) and β-CD (c);
FIGs. 4 and 5: Matrix-assisted laser desorption ionization time-of-flight mass spectrometry (MALDI-TOF MS) analysis results of β-CD (FIG 4) and the inventive dimeric β-CD obtained in Example 1 (FIG 5) (the inserts show the mass ion spectra of peak regions); and
FIG 6: Photofluorescence results showing the fluorescence quenching of the inventive dimeric β-CD obtained in Example 1 at a concentration of 0 M (a), 6.0 x 10⁻⁵ M (b), 1.3 x 10⁻⁴ M (c), 2.3 x 10⁻⁴ M (d), 3.5 x 10⁻⁴ M (e), 4.9 x 10⁻⁴ M (f) or 6.3 x 10⁻⁴ M (g) (the insert shows the UV/Vis absorption spectrum of RhB).

### Detailed Description of the Invention

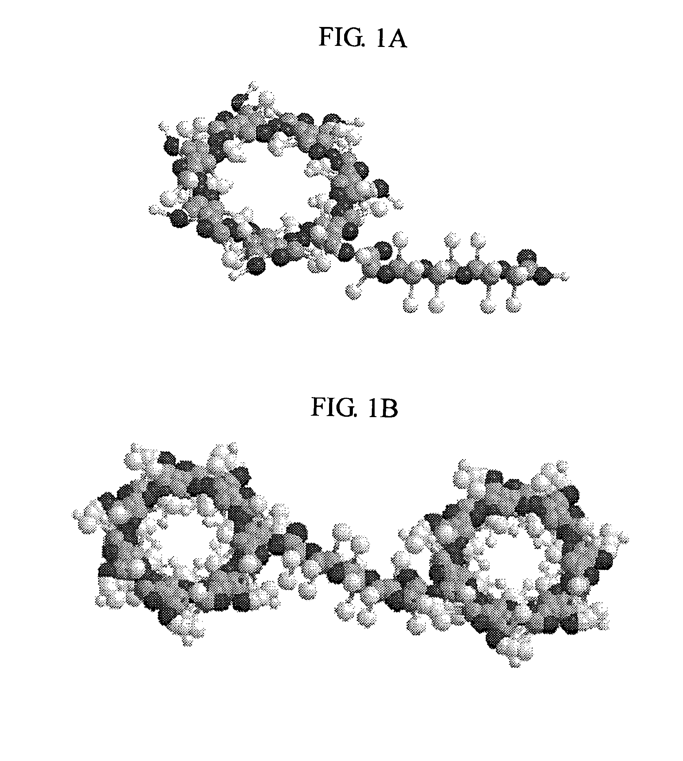

The conjugate of cyclodextrin (CD) and poly(oxyethylene) (POE) of the present invention may be a CD molecule linked with one POE (monomeric CD), as shown in FIG 1A; a polymer comprising such monomeric CD repeating units (polymeric CD); or two CD molecules linked by one POE (dimeric CD), as shown in FIG 1B, and in most cases, the inventive CD conjugate exist as a mixture thereof

In the CD conjugate of the present invention, the CD may be α-CD, β-CD, γ-CD or η-CD.

The CD conjugate of the present invention can accommodate a variety of guest molecules into its CD hydrophobic cavity through van der Waals force and hydrophobic interaction between the CD cavity and guest molecule. Further, the inventive CD conjugate can form diverse supramolecular structures such as linear-polymer, dendrimer and star-polymer comprising the monomeric or dimeric CD as repeating unit, while the monomeric or dimeric CD may be modified by adjusting the length of the POE spacer, which preferably has a molecular weight ranging from 88 to 10,000.

The inventive CD conjugate may be prepared by a process comprising (i) reacting poly(ethyleneglycol) bis(carboxymethyl) ether with thionyl chloride or oxalyl chloride under a nitrogen atmosphere to obtain a poly(oxyethylene) bis-acid chloride derivative; and (ii) reacting the acid chloride derivative obtained in step (i) with cyclodextrin in a solvent.

In step (i), thionyl chloride or oxalyl chloride may be employed in an amount ranging from 20 to 35 ml, preferably from 30 to 35 ml based on 1 g of poly(ethyleneglycol) bis(carboxymethyl) ether, and the reaction may be conducted at a temperature raging from 70 to 80°C, preferably from 75 to 80°C for 5 to 8 hours, preferably 6 to 7 hours.

In step (ii), the CD, which may be selected from the group consisting of α-CD, β-CD, γ-CD and η-CD, is employed in amounts of 1 to 10 folds, preferably 3 to 4 folds by weight based on the weight of the POE bis-acid chloride derivative, and the solvent may be selected from the group consisting of anhydrous N,N-dimethylformamide (DMF), pyridine, acetonitrile, ethyl ether, dimethyl sulfoxide and water, depending on the solubility of the educts and the coupling method. Step (ii) may be conducted at a temperature ranging from 0 to 150°C for 2 hours to 2 weeks with slowly heating.

Further, the inventive CD conjugate may be prepared by a process comprising directly reacting poly(ethylene glycol) bis(carboxymethyl) ether with cyclodextrin in the presence of a carboxyl group-activating coupling agent.

In the reaction, the carboxyl group-activating coupling agent may be selected from the group consisting of carbonyl diimidazole and carbodicyclohexylcarboiimide, and the carboxyl group-activating coupling agent or poly(ethylene glycol) bis(carboxymethyl) ether may be employed in an amount ranging from 0.1 to 2 moles, preferably 0.5 mole based on cyclodextrin. The reaction may be conducted in a solvent selected from selected from the group consisting of anhydrous N,N-dimethylformamide (DMF), pyridine, acetonitrile, ethyl ether, dimethyl sulfoxide and water at a temperature ranging from 0 to 150°C.

The CD conjugate obtained by the inventive process may be a mixture composed of mainly dimeric CD together with some monomeric CD and polymeric CD, and the composition of the mixture can be controlled by modifying the relative amounts of reactants, reaction temperature, and reaction time in the inventive method.

The CD conjugate prepared by the present invention can form biocompatible supramolecular structures having excellent inclusive property and structural diversity, and thus, can be beneficially employed in various fields such as drug delivery, food and flavors, cosmetics, packing, textiles, separation processes, environment protection, fermentation and catalysis.

The following Examples are given for the purpose of illustration only and are not intended to limit the scope of the invention.

### Example 1: Preparation of the inventive cyclodextrin conjugate having a poly(oxyethylene) spacer

A mixture of 1 g (4 mmol) of poly(ethylene glycol) bis(carboxymethyl) ether (molecular weight: 250, Aldrich) and 30 ml of thionyl chloride was refluxed under a nitrogen atmosphere for 6 hours, and the unreacted thionyl chloride was removed from the reaction mixture. 1.033 g of poly(oxyethylene) bis-acid chloride derivative was obtained as a deep-yellow liquid (yield 89.9%).

32 mg (0.14 mmol) of poly(oxyethylene) bis-acid chloride was dissolved in 13 ml of anhydrous DMF, and added thereto 500 mg (0.44 mmol) of dried β-cyclodextrin (Aldrich) dissolved in 14 ml of anhydrous pyridine. The resulting mixture was stirred for 9 hours over an ice bath, warmed up to room temperature, and stirred at that temperature for two days to obtain a light-yellow solution. The solution was subjected to vacuum evaporation to remove the solvent, and the residue was poured into 100 ml of acetone. The white solid formed was washed several times with 100 ml portion of acetone. The residual acetone was removed in vacuo, to obtain the title compound as a light-yellow solid.

The compound mixture thus obtained was subjected to column chromatography using a Sephadex G-25 column and deionized water as an eluent to separate therefrom monomeric, dimeric and polymeric CD conjugates. Each of the isolated CD derivatives was gravimetrically analyzed. As a result, it was found that the product was composed of 37% (399 mg) dimeric CD, the remainder being monomeric CD, polymeric CD, and residual starting CD.

### Analysis of starting and synthesized compounds

### (1)FT-IR spectra

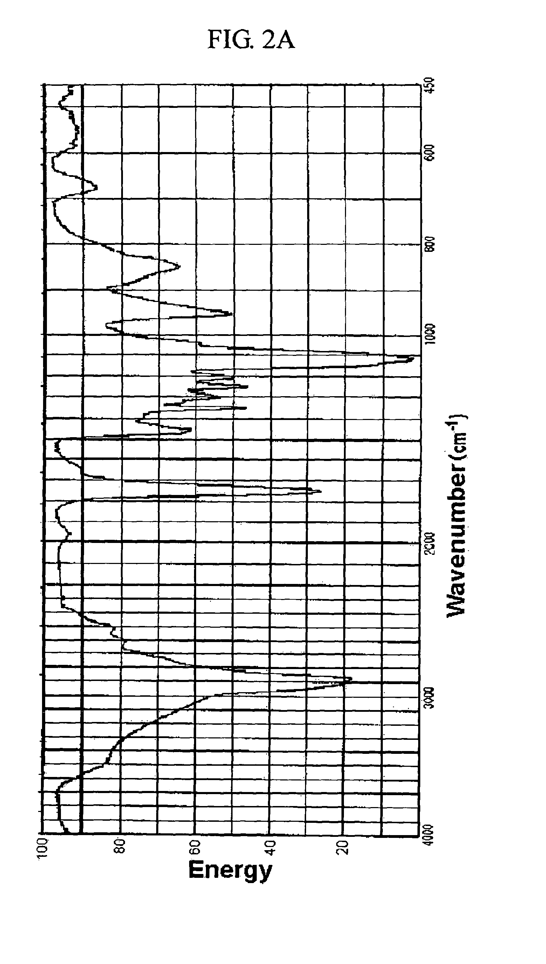

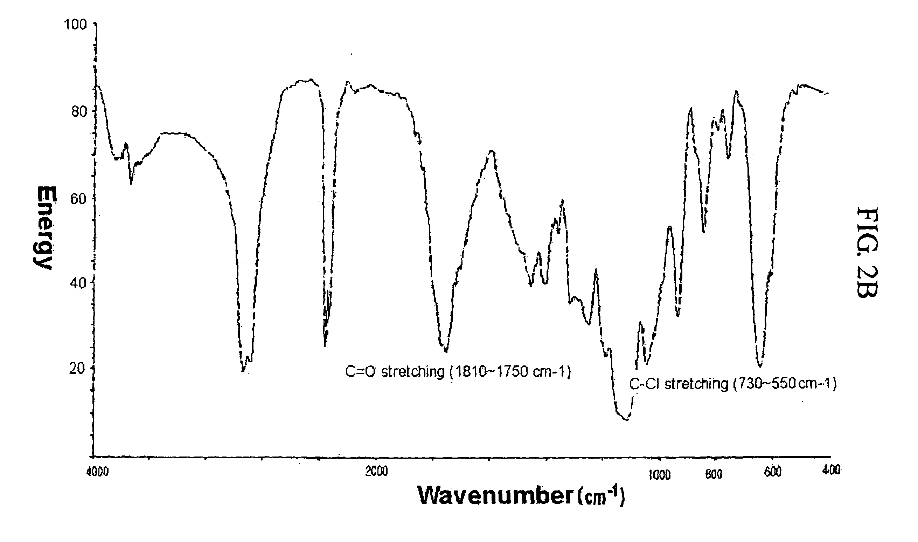

FT-IR spectra of poly(ethyleneglycol) bis(carboxymethyl) ether and poly(oxyethylene) bis-acid chloride derivative are shown in FIGs. 2A and 2B, respectively.

The figures show that specific peaks for acid chloride, C=O stretching (1810~1750 cm⁻¹) and C-Cl stretching (730~550 cm⁻¹), are absent in the spectrum of poly(ethyleneglycol) bis(carboxymethyl) ether but present in the spectrum of poly(oxyethylene) bis-acid chloride derivative.

### (2) TGA

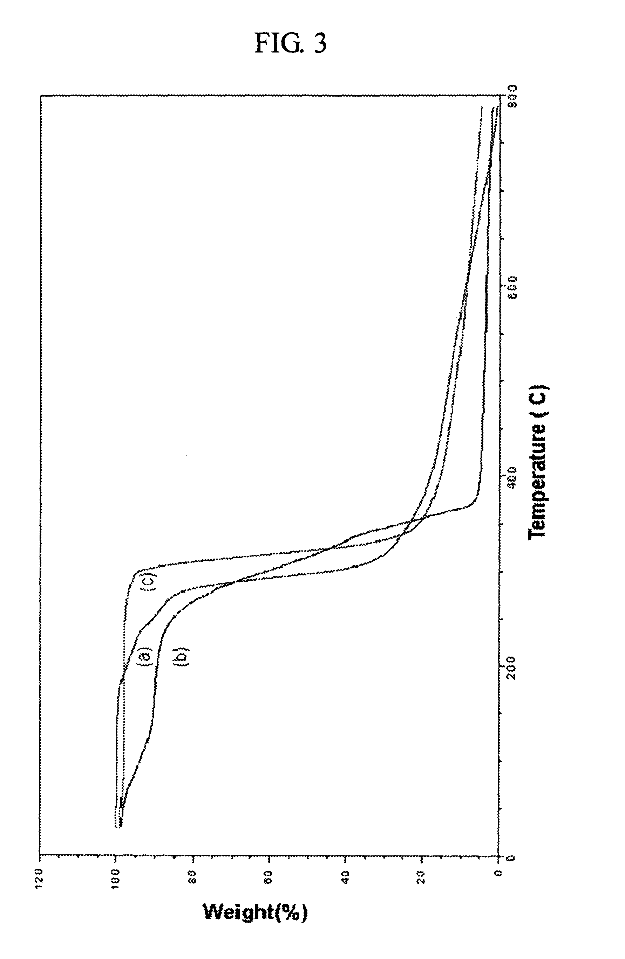

β-Cyclodextrin (a), poly(oxyethylene) bis-acid chloride derivative (b), and the dimeric β-CD conjugate obtained in Example 1 (c) were subjected to TGA (thermogravimetric analysis) in order to study their thermal stabilities, and the results are shown in FIG 3.

As shown in FIG 3, the pristine β-CD has the highest thermal stability and the poly(oxyethylene) bis-acid chloride derivative has the lowest, among those three compounds, and as expected, the thermal stability of the inventive dimeric β-CD was intermediate between the above-mentioned two.

### (3) MALDI-TOF MS

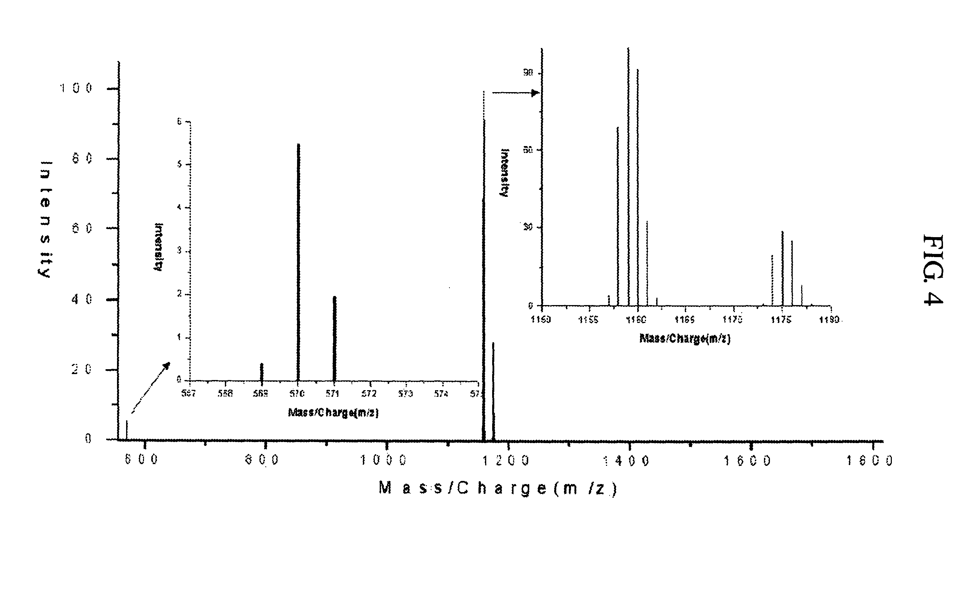

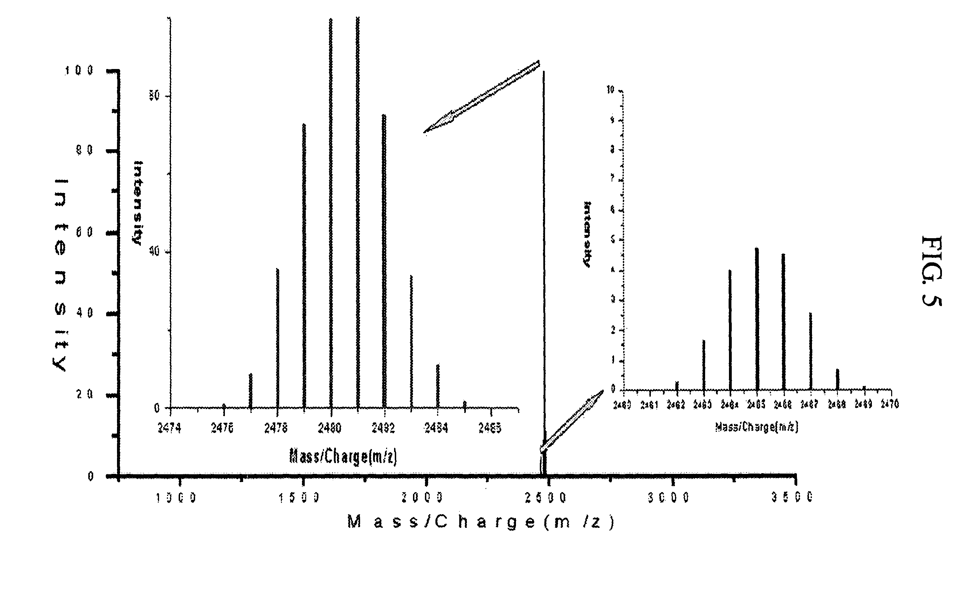

The pristine β-CD and the dimeric β-CD conjugate obtained in Example 1 were subjected to MALDI-TOF MS (matrix material: cyano-4-hydroxycinamic acid (CHCA)), and the results are shown in FIGs. 4 and 5, respectively.

As shown in FIGs. 4 and 5, the molecular mass of β-CD is 1135 and that of the inventive β-CD conjugate is 2480, which indicates that the inventive dimeric β-CD is composed of two β-CDs and one POE spacer.

### (4) Measurement of inclusive property

The inclusive property of the inventive CD conjugate was evaluated by performing fluorescence quenching using a 20% acetonitrile aqueous solution containing 1.0 x 10⁻⁶ M Rhodamine B (RhB), as follows.

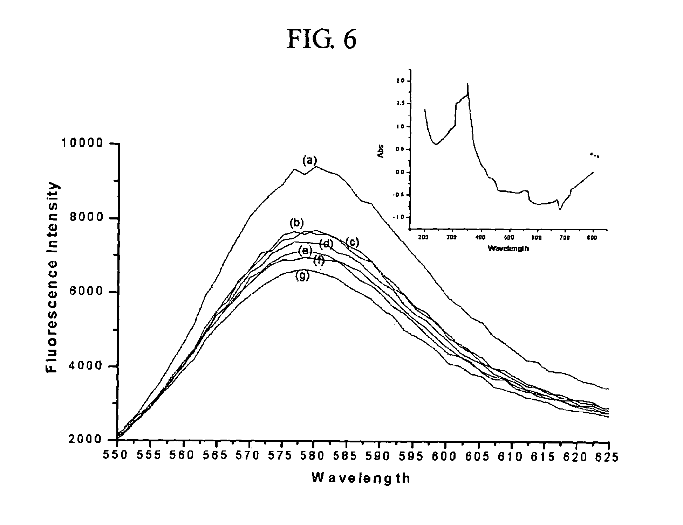

The dimeric β-CD conjugate obtained in Example 1 was added to the RhB acetonitrile solution to a concentration of 0 M (a), 6.0 x 10⁻⁵ M (b), 1.3 x 10⁻⁴ M (c), 2.3 x 10⁻⁴ M (d), 3.5 x 10⁻⁴ M (e), 4.9 x 10⁻⁴ M (f) or 6.3 x 10⁻⁴ M (g), and each test solution thus obtained was subjected to UV/Vis spectrometry to obtain the photofluorescence spectra shown in FIG 6.

FIG 6 shows that the degree of fluorescence quenching of the test solution is dependent on the concentration of the dimeric β-CD, and this quenching phenomenon is due to the equilibrium shift of RhB from the hydrophilic and fluorescent carboxylate ion form to the hydrophobic and nonfluorescent lactone form produced by the host-guest interaction with the dimeric β-CD.

As can be seen from the above, the novel CD conjugate of the present invention is composed of non-toxic compounds to form highly biocompatible supramolecular structures exhibiting significant inclusive property and structural diversity, and thus, can be beneficially employed in various fields such as drug delivery, food and flavors, cosmetics, packing, textiles, separation processes, environment protection, fermentation and catalysis.

While the invention has been described with respect to the specific embodiments, it should be recognized that various modifications and changes may be made by those skilled in the art to the invention which also fall within the scope of the invention as defined by the appended claims.

## Claims

1. A cyclodextrin (CD) conjugate having a poly(oxyethylene) (POE) moiety as a spacer.

2. The cyclodextrin conjugate of the claim 1, which is in the form of a CD molecule linked with one POE (monomeric CD), a polymer comprising the monomeric CD repeating units (polymeric CD), two CD molecules linked by one POE (dimeric CD), or a mixture thereof

3. The cyclodextrin conjugate of the claim 1, wherein the CD is selected from the group consisting of α-CD, β-CD, γ-CD and η-CD.

4. The cyclodextrin conjugate of the claim 1, wherein the poly(oxyethylene) has a molecular weight ranging from 88 to 10,000.

5. A method for preparing the cyclodextrin conjugate of the claim 1 comprising: (i) reacting poly(ethylene glycol) bis(carboxymethyl) ether with thionyl chloride or oxalyl chloride to obtain a poly(oxyethylene) bis-acid chloride derivative; and (ii) reacting the poly(oxyethylene) bis-acid chloride derivative obtained in step (i) with cyclodextrin.

6. The method of the claim 5, wherein the cyclodextrin is selected from the group consisting of α-CD, β-CD, γ-CD and η-CD.

7. The method of the claim 5, wherein the cyclodextrin is employed in an amount ranging from 1 to 10 folds by weight based on the weight of the poly(oxyethylene) bis-acid chloride derivative.

8. The method of the claim 5, wherein step (ii) is conducted in a solvent selected from the group consisting of anhydrous N,N-dimethylformamide, pyridine, acetonitrile, ethyl ether, dimethyl sulfoxide and water.

9. The method of the claim 5, wherein step (ii) is conducted at a temperature ranging from 0 to 150°C.

10. A method for preparing the cyclodextrin conjugate of the claim 1 comprising reacting poly(ethylene glycol) bis(carboxymethyl) ether with cyclodextrin in the presence of a carboxyl group-activating coupling agent.

11. The method of the claim 10, wherein the cyclodextrin is selected from the group consisting of α-CD, β-CD, γ-CD and η-CD.

12. The method of the claim 10, wherein the carboxyl group-activating coupling agent is selected from the group consisting of carbonyl diimidazole and carbodicyclohexylcarboiimide.

13. The method of the claim 10, wherein the carboxyl group-activating coupling agent or poly(ethylene glycol) bis(carboxymethyl) ether is employed in an amount ranging from 0.1 to 2 moles based on cyclodextrin.

14. The method of the claim 10, wherein the reaction is conducted in a solvent selected from selected from the group consisting of anhydrous N,N-dimethylformamide (DMF), pyridine, acetonitrile, ethyl ether, dimethyl sulfoxide and water.

15. The method of the claim 10, wherein the reaction is conducted at a temperature ranging from 0 to 150°C.
